# EUROPEAN PATENT SPECIFICATION

(11) **EP 1 360 642 B1**
(45) Date of publication and mention of the grant of the patent: **04.08.2004**
(21) Application number: 02701461.2
(22) Date of filing: 07.02.2002
(51) Int. Cl.: G06K 11/12, G06K 11/06, G06K 11/08, G06K 11/16, B25J 19/02, G01L 1/20

(54) **DEVICE FOR DETECTING THE SHAPE AND/OR POSITION OF OBJECTS, AND/OR FOR GRIPPING AND LIFTING OBJECTS**
GERAET ZUM ERKENNEN DER FORM UND/ODER POSITION VON GEGENSTAENDEN, UND/ODER ZUM GREIFEN UND ANHEBEN VON GEGENSTAENDEN
DISPOSITIF DE DETECTION DE LA FORME ET/OU DE LA POSITION D'OBJETS, ET/OU DE PREHENSION ET D'ELEVATION D'OBJETS

(30) Priority: 09.02.2001 HU 0100663
(43) Date of publication of application: 12.11.2003
(73) Proprietor: Pali, Jeno, 2100 Gödöllo (HU)
(72) Inventor: Pali, Jeno, 2100 Gödöllo (HU)
(74) Representative: Sari, Tamas Gusztav
(86) International application number: PCT/HU2002/000010
(87) International publication number: WO 2002/063549

(56) References cited:
- US-A- 4 014 217
- US-A- 4 555 953
- US-A- 5 060 527
- US-A- 6 002 389

## Description

The invention relates to a device for detecting the spatial pattern on surfaces and/or the shape of objects, and/or the local displacements on surfaces, and/or for slip-free gripping and lifting of an object applying an adequate compression force thereon. The invention can be used for providing artificial touch sensing and gripping devices capable of detecting both the direction, extent, duration of the compression force affecting the contacting/gripping surface of the devices and the three-dimensional deformation caused by it, transforming those into electric signals, and which is capable of the slip-free gripping and lifting of objects with an adequate compression force.

Both basic and industrial research experiments have been carried out for a long time for creating touch sensing and gripping devices operating similarly to human skin: they should be able to detect the spot, extent, direction and duration of the compression force acting on their surfaces and to generate signals for the processing computer which signals make both the recognition of spatial pattern on surfaces (textures) and/or shape of objects and the discrimination among distinct textures and shapes possible. Similarly, gripping devices should be able to handle objects with adequate compression force without any slippage or crunch.

The best known touch sensing devices are the resistive-membrane position sensors which have been used in keyboards for many years.

Other touch sensing devices are operating with the change in capacitance between a conductive reference layer and a matrix of conductive traces separated by a deformable insulating layer due to the two capacitive conductive surfaces being closer to each other by pressing with a stylus on the surface of the touch-pad (Kasser, US 6,002,389).

In an other embodiment of touch sensing devices, the insulating layer may exhibit the piezoresistive-effect: the electrical resistance of the said layer containing conductive fillers (e.g. finely divided particles of metal or carbon) or consisting of electrically conductive cellular foam material varies in response to deformation (Burgess, US 5,060,527). If the layer is sandwiched between two conductive plates, the current flowing between them will vary according to the deformation of the insulating layer.

Similarly, the tactile pick-up of Lagasse et al (US 4,014,217) possesses a continuous outer layer of a material which has a variable electrical conductivity as a function of its state of compression and a second layer comprising a matrix of measuring electrodes. When compressed by an object, the electrodes lying at the adjacent points of the deformation of the outer layer detect the changes in the conductivity.

Dario et al (US 4,555,953) have developed a composite, multilayered touch sensing device based on the ferroelectric properties of polymeric materials (e.g. polymer polyvinylidene fluoride). The said device comprises one superficial (first ferroelectric polymer transducer) and one deep (second ferroelectric polymer transducer) sensing layers, separated by an intermediate layer of compliant material, interposed between them. Both the said superficial and deep sensing layers comprise films of ferroelectric polymer material and matrix-like arrays of electrodes. The touch sensing device of Dario is believed to form the most relevant piece of prior art relating to the present invention, and the claims are delimited against this document.

The aforementioned touch sensing devices are lacking both the complexity and the modular organization of the ridged skin of the human fingertips.

The object of the present invention is to provide a device capable of detecting the spatial pattern on surfaces and/or the shape of objects, and/or the local displacements on surfaces, and/or of slip-free gripping and lifting of an object applying an adequate compression force thereon, i.e. to create a touch sensing and gripping device capable of detecting the compression acting on it and spreading in it, then to convert it into electric signals which can be processed by a computer for shape and pattern recognition, and which is capable of the slip-free gripping and lifting of an object applying an adequate compression force thereon.

Accordingly the invention provides a device capable of detecting the spatial pattern on surfaces and/or the shape of objects, and/or the local displacements on surfaces, and/or of slip-free gripping and lifting of an object applying an adequate compression force thereon containing deformable, resilient sensor carrier layers and deformable intermediate layers therebetween, wherein the individual intermediate layers are more easily deformable (malleable) than the adjacent sensor carrier layers. Geometrical formations are provided on the surfaces of the layers forming convex surface parts on one of the surfaces of the layers, and concave surface parts on the other surfaces thereof. Exceptions are the external surfaces of the exterior sensor carrier layers. Each of the convex surface parts of the sensor carrier layers has the same shape and size as the corresponding concave surface part of the adjacent sensor carrier layer and the convex surface part is fitted into the concave surface part with the intermediate layer in between. Sensors are connected to the convex surface parts of the sensor carrier layers and to the concave surface parts of the adjacent sensor carrier layers in pairs for detecting the extent of the displacement between the convex and concave surface parts, and/or the rate of this displacement, and/or the extent of tension or compression resulting from this displacement, and the sensors are provided with means for carrying the output signals thereof.

The geometrical formations are preferably polygon-base pyramids, truncated pyramids, cones, conical frusta or hemispheres. There may be a sensor connecting the convex and the concave surface parts fitted into one another at a point of the geometrical formations being farthest from the plane of the corresponding sensor carrier layer and/or there may be at least three sensors connecting the convex and the concave surface parts fitted into one another at points of the geometrical formations other than the point being farthest from the plane of the corresponding sensor carrier layer.

In another preferred embodiments, the geometrical formations are tetrahedrons or square-base pyramids. There may be a sensor connecting the convex and the concave surface parts fitted into one another at the vertices of the geometrical formations and/or there may be at least one sensor connecting the convex surface parts and the concave surface parts fitted into one another at each of the planes of the geometrical formations.

There may be convex and concave surface parts formed by geometrical formations of different shapes or sizes on the sensor carrier and the intermediate layers and the density of the convex and the concave surface parts may also be varying on them. Accordingly regions of different characteristics (e.g. different resolution or sensitivity) may be provided.

The external surface of one of the exterior sensor carrier layers is preferably a contacting surface for connecting to the object to be touched or gripped. The contacting surface may be provided with bristles or ribs separated by grooves, or the contacting surface may be formed by a soft, malleable, smooth contacting layer fixed to the exterior sensor carrier layer. The external surface of the other exterior sensor carrier layer is preferably a base surface for connecting to a support surface on which the device is placed.

Preferably the thickness of the sensor carrier layers increases from the contacting surface towards the base surface, while the thickness of the intermediate layers decreases. Preferably the stiffness of the sensor carrier layers and the viscosity of the intermediate layers increase from the contacting surface towards the base surface.

The sensors are preferably Hall-sensors, potentiometers, optical sensors, magnetic sensors, capacity-type sensing elements, piezoelectric transducers or sensing elements made of conductive rubber.

Thus the sensors are detecting the extent of displacement between the opposing convex and concave surface parts of the plates constituting the adjacent sensor carrier layers, the rate of this displacement, or the extent of tension or compression originating from this displacement, then it is transferred into a computer which calculates the direction (resultant vector of displacements between pairs of opposing convex and concave surface parts) of the compression force acting on a given spot of the contacting surface of the device, its magnitude (how deep the deformation is), its rate (how fast the deformation spreads into the layers lying under each other) and its duration (how long deformation lasts) from the displacements of the surface parts (geometrical formations) relative to each other. An "enveloping surface" can be drawn to the displacements between the adjacent surface parts of the sensor carrier layers in the device by any high-tech computer, which provides the three-dimensional imprint of the pattern on surfaces and/or the shape of objects by which the contacting surface of the device has been deformed.

The plate-like sensor carrier layers overlying each other are separated by liquid, semiliquid or solid, but malleable intermediate layers forming some kind of "hydrostatic framework", just like in cases of e.g. soft-stalked plants or worms. The thinner the consistence of the material of the intermediate layers separating the sensor carrier layers, and/or the more the elasticity/flexibility of the sensor carrier layers, and/or the higher the density of the geometrical formations on the layers, the easier is to deform the layers near the contacting surface, resulting in the increase of the sensitivity of the touch sensing with the device according to the invention.

Furthermore, the exterior sensor carrier layer forming the contacting surface may contain thermal sensors detecting the temperature of the object being touched or gripped and/or chemical sensors detecting for example the pH. These sensors may be arranged on the contacting surface or close thereto.

The invention will be described further, by way of example, with reference to the accompanying drawing wherein:
figure 1 shows the characteristic cross-section of a first embodiment of a device suitable for detecting the spatial pattern on surfaces, and/or the local displacements on surfaces, and/or for slip-free gripping and lifting of an object applying an adequate compression force thereon,
figure 2 shows the characteristic cross-section of a second embodiment of a device which is capable of detecting the shape and size of objects, and
figure 3 shows the characteristic cross-section of a third embodiment of the invention, which is capable of the slip-free, stable gripping and transporting of large objects.

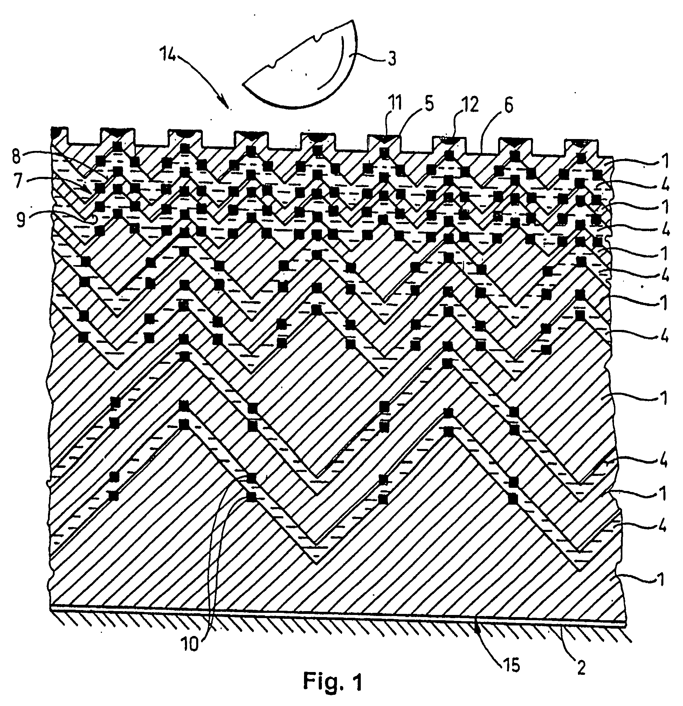

The first embodiment of the invention can be used as an artificial skin covering the hands (particularly the fingers) of human prostheses or robotic limbs (see figure 1).

The device contains a medium number, i.e. in this case of seven plate-like sensor carrier layers 1; from these, one of the exterior sensor carrier layers 1 forms a contacting surface 14 getting into direct contact with an object 3 to be touched or gripped, whereas the other exterior sensor carrier layer 1 forms a base surface 15 fitted to a support surface 2 (fingers of the prosthetic or robotic hand). Sensor carrier layers 1 are separated from each other by intermediate layers 4.

Ribs 5 of a height of several tenths of a millimetre and a breadth of 0,1-1 mm, and grooves 6 of the same order of magnitude in their breadth separating the ribs 5 are provided on the external surface of the exterior sensor carrier layer 1 contacting with object 3 to be touched or gripped. On the one hand, they prevent the object 3 from slipping relative to the contacting surface 14 when the object 3 is gripped and on the other hand, in case of pattern recognition, ribs 5 in the device forming an artificial skin may be displaced easier (with lower threshold) than it would be if detecting moving edges on a flat surface, i.e. the device works similarly to the ridged skin of human fingertips. Instead of ribs 5 and grooves 6, the contacting surface 14 may be provided with bristles, too.

To provide additional information, the exterior sensor carrier layer 1 forming the contacting surface 14 contains thermal sensors 11 detecting the temperature of the object 3 being touched or gripped and chemical sensors 12 detecting for example the pH. These thermal sensors 11 and chemical sensors 12 are arranged close to the contacting surface 14.

Geometrical formations 7 of pyramids are provided both on the sensor carrier layers 1 and the intermediate layers 4 forming convex surface parts 8 on one of the surfaces of the sensor carrier layers 1 and of the intermediate layers 4, and concave surface parts 9 on the other surfaces of the sensor carrier layers 1 and of the intermediate layers 4, except for the external surfaces of the exterior sensor carrier layers 1. The convex surface parts 8 of the sensor carrier layers 1 are fitted into the corresponding concave surface parts 9 of the adjacent sensor carrier layer 1 with the intermediate layer 4 in between. In this embodiment, the geometrical formations 7 fill in completely the surfaces of each sensor carrier layers 1 and intermediate layers 4. The pyramids forming the convex surface parts 8 and the concave surface parts 9 of sensor carrier layers 1 near the contacting surface 14 are preferably smaller than the ones being far therefrom. In other words the density of the convex surface parts 8 and the concave surface parts 9 is changing in the device. Thus the plates forming the sensor carrier layers 1 near the contacting surface 14 can detect the spatial pattern on the surface of the touched object 3 with greater resolution, i.e. the sensitivity of touch sensing is higher. This way the resolution of the device is changing vertically, however the size and/or the density of the geometrical formations 7 forming the convex surface parts 8 and the concave surface parts 9 may change horizontally, too, resulting in more and less sensitive areas of the artificial skin formed by the device.

Sensors 10 are connected to the convex surface parts 8 and concave surface parts 9 of adjacent sensor carrier layers 1 fitting into one another with the intermediate layer 4 in between. Sensors 10 are arranged on the surfaces of two adjacent sensor carrier layers 1 with the geometrical formations 7 of identical shape and size both at the opposite planes and vertices of each geometrical formations 7 detecting their displacement relative to each other. The signals originating from these sensors 10 are transferred by a wiring (not shown) embedded into the plates forming the sensor carrier layers 1.

The plates forming the sensor carrier layers 1 of the device are preferably made of some flexible, elastic plastic material or rubber. At going from the contacting surface 14 to the direction of the base surface 15, their thickness increases, but it remains in the order of magnitude of 0.1-1 mm, and parallel to this, the sensor carrier layers 1 become harder and harder, e.g. they are made of more and more cured rubber. As to the thickness of intermediate layers 4 made of some liquid, gel-like material, eventually of soft plastic or rubber, the situation is the opposite. The thickness of these decreases to the direction of the base surface 15, and at the same time, going into this direction, more and more viscous materials can be used as intermediate layer 4.

Thus the total thickness of the touch sensing device forming artificial skin is several millimetres. Geometrical formations 7 formed on sensor carrier layers 1 are preferably pyramids with edges in the millimetre range, both on the side-planes and vertices emerging from the surfaces of sensor carrier layer 1 of which Hall-sensors operating on the basis of electromagnetic induction are arranged. In this embodiment pyramids of three different sizes form the convex surface parts 8 and the concave surface parts 9 in the device.

In the device operating as artificial skin, the soft, flexible plates forming the sensor carrier layers 1 and the intermediate layers 4 of low viscosity make the accurate imaging of fine spatial pattern on the surface of the object 3 possible in the part of the artificial skin close to object 3. At the same time, it is not the task of the artificial skin covering an artificial hand to detect the three-dimensional shape of the object 3 to be touched or gripped; it would not be able to do this, as the artificial skin cannot be so thick that a bigger object 3 could be sunk totally into it (a solution for this problem will be dealt with in the next embodiment). The recognition of shape (stereognosis) in human touch is also the result of the operation of the higher order cortices, in which, in addition to fine, discriminative touch, a greater role is played by crude, non-discriminative touch and by the position sense (proprioception) of the fingers and finger-joints.

However, the device forming artificial skin according to the invention and covering the fingers of prostheses or robotic limbs, similarly to human skin, should be able to perform a further task: slip-free gripping and lifting of an object 3 applying an adequate compression force thereon, since - in the case of a prosthesis - in absence of this function, the human being is not capable of performing one of the most characteristic human performance: manipulation of objects. At the beginning, the grip on object 3 is relatively weak. The initial, weak grasp is not enough for a stable holding of the object 3, thus the surface of the artificial skin (i.e. the first sensor carrier layer 1) connecting with the surface of the object 3 starts to slip, together with the object 3. Then the underlying plates forming the second, third, and so on sensor carrier layers 1 start also to slip relative to each other. At the same time, the tips of the convex surface parts 8 constituting the geometrical formations 7, and the tips of the opposite concave surface parts 9 of the adjacent sensor carrier layer 1 move away from each other activating thereby sensors 10 connecting the vertices (and also the planes) of the convex surface parts 8 and concave surface parts 9 formed by geometrical formations 7. As a feedback, continuous computer signal processing increases the grasp of the prosthesis or robotic limb. As a result of this increasing compression, and due to the given arrangement of geometrical formations 7 and to the low viscosity of intermediate layers 4, plates forming the sensor carrier layers 1 near the object 3 slip back, and are pressed to each other. If object 3 is not kept strongly enough yet, these sensor carrier layers 1 near the object 3 will move jointly relative to the deeper lying plates forming sensor carrier layers 1. Since the geometrical formations 7 of deeper sensor carrier layers 1 get larger and larger and the thickness and stiffness of sensor carrier layers 1 increase, and the liquid constituting the intermediate layers 4 gets more and more viscous and gel-like, the lateral displacement of deeper lying sensor carrier layers 1 relative to each other gets smaller and smaller. Preferably, the system is optimised so that unitary displacement of deeper lying sensor carrier layers 1 relative to each other is compensated by a greater increment in the gripping force than the lateral displacement of the same extent in sensor carrier layers 1 near the object 3, until the holding of the object 3 does not cause any further displacement in the artificial skin.

Sensors 10 connecting the tips of the convex surface parts 8 of a sensor carrier layer 1 and the tips of the corresponding concave surface parts 9 of the adjacent sensor carrier layer 1 detect the slip of sensor carrier layers 1 relative to each other, their moving away, independently of the direction of the displacement, whereas the sensors 10 arranged on the planes of an individual geometrical formation 7 of a sensor carrier layer 1, as a functional unit, are capable of detecting the direction of the compression force acting on the artificial skin in addition to the nearing and moving away of the geometrical formation 7 of the adjacent sensor carrier layer 1 by calculating the resultant vector of the displacements between individual pairs of planes.

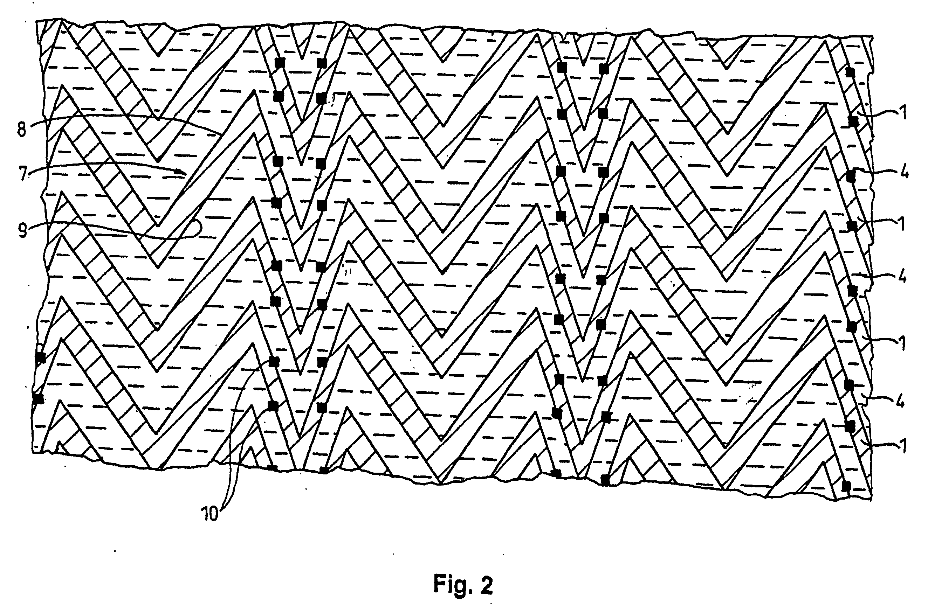

Figure 2 shows the characteristic cross-section of a few sensor carrier layers 1 and intermediate layers 4 of a second embodiment of the invention, which is capable of shape and size recognition in the range from tenths of millimetres to several centimetres. (For example, for producing three-dimensional fingerprints and palmprints, or e.g. in vegetable and fruit sorting systems for the imaging of the accurate, three-dimensional shape and size.)

In this case it is important that the given object 3 should be able to sink totally into the device according to the invention performing artificial touch. This is possible, if each sensor carrier layer 1 is equally thin and to a great extent flexible (e.g. only slightly cured rubber), and intermediate layers 4 are thicker and formed of low viscosity fluids (e.g. water). The number of sensor carrier layers 1 and intermediate layers 4 depends on the size of the objects 3 to be imaged spatially; this number may be even several hundreds. The geometrical formations 7 of sensor carrier layers 1 are tetrahedrons with edges of several tenths of a millimetre. The thickness of both sensor carrier layers 1 and intermediate layers 4 falls into the same size range. The base planes of the tetrahedrons fill in completely the surfaces of the sensor carrier layers 1 and the intermediate layers 4 and one of the edges of the tetrahedrons and the mid-line of the opposite planes thereof fit in the intersecting plane.

The shape of the object 3 is reconstructed from signals originating from sensors 10 detecting optimum-sized displacement: sensor carrier layers 1 near the object 3 are compressed to each other by object 3 being pressed into the device, and sensors 10 situated here provide too large signal, whereas owing to the sinking of sensor carrier layers 1 near the object 3, displacements are caused in a much larger region than the real volume of the object 3, however, these displacements are getting smaller when moving off from object 3. The detecting range of sensors 10 providing the real surface, size, and shape of the object 3 can be determined by calibration.

In this embodiment, the contacting surface 14 of the device should be perfectly smooth (without any grooves, ribs or bristles). The whole device can be placed onto a workbench, as a support surface 2.

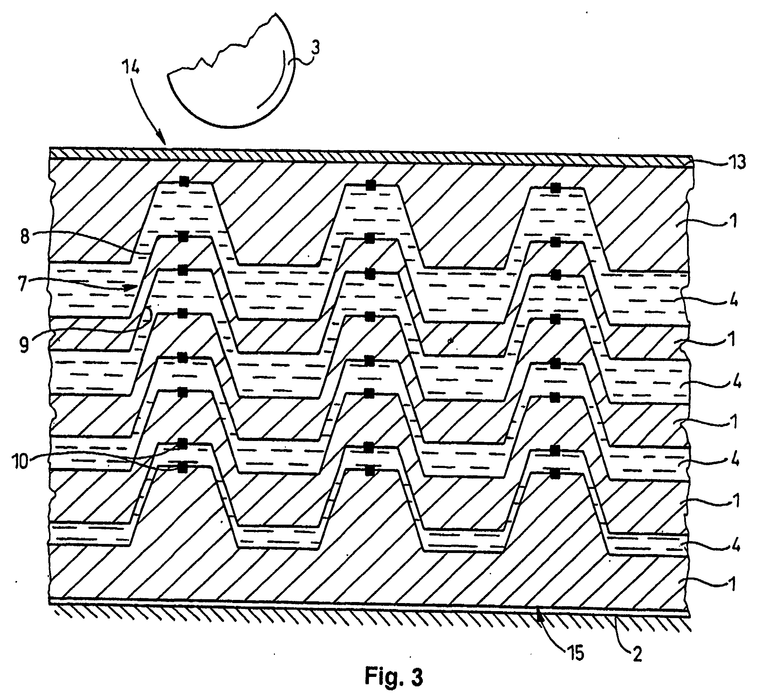

Figure 3 shows the characteristic cross-section of a third embodiment of the invention which is capable of the slip-free, stable gripping and transporting of large objects 3.

In this case, the device is expediently built of e.g. five flexible, but highly steady plates as sensor carrier layers 1. The material of these plates may be strongly cured rubber, highly polymerised plastics or Plexiglas. Intermediate layers 4 may contain of semiliquid or gel-like materials. The thickness of the plates forming the sensor carrier layers 1 is increased while that of the intermediate layers 4 is decreased from the contacting surface 14 towards the base surface 15 as in the first embodiment of the invention. Similarly, the stiffness of the sensor carrier layers 1 and the viscosity of the intermediate layers 4 is increased in this direction.

The geometrical formations 7 of sensor carrier layers 1 are conical frusta with diameters of the base surfaces in the order of magnitude of 1-10 cm, whereas their height falls in the centimetre range. The thickness of the plates forming sensor carrier layers 1 and that of intermediate layers 4 are in the same range.

In this embodiment the base planes of the geometrical formations 7 do not fill in completely the surfaces of the sensor carrier layers 1 and the intermediate layers 4, the distance of the centre points of the base surfaces of two adjacent conical frusta is greater than the diameter thereof.

All of the convex surface parts 8 and the concave surface parts 9 are formed by geometrical formations 7 of the same size and shape and they are arranged in vertically ordered column-like series.

Sensors 10 are connecting the top planes of the convex surface parts 8 and the concave surface parts 9 of adjacent sensor carrier layers 1 forming the conical frusta. Additional sensors may be arranged on the curved surface of the conical frusta, too. These sensors 10 may be e.g. potentiometers, but optical sensors, magnetic sensors, capacity-type sensing elements, piezoelectric transducers or sensing elements made of conductive rubber containing an elastic polymer resin and some conductive additive can also be created and applied. For carrying the output signals of sensors 10, wiring may be used, but radiofrequency transmitters, or optical fibres embedded into the device can also be imagined.

The surface contacting with object 3 to be moved, the contacting surface 14, is formed by a contacting layer 13 of soft, well-deformable (malleable), but elastic slightly cured rubber, fixed to the topmost sensor carrier layer 1, into which the given object 3 can sink to a small extent, thus the compression force acting onto object 3 can better spread on the surface of the object 3, and due to larger friction, the object 3 is connected to the device in a more stable manner.

Artificial skin covering the prosthetic or robotic hand should be suitable for both touch sensing and slip-free holding of the object 3. In a device serving for shape and size recognition the capability for holding object 3 does not play an important role, whereas in the third embodiment, in the power machine, by gripping the objects 3, the recognition of the shape and surface unevenness of the large-sized, large mass object 3 is not an important feature.

The advantage of the device according to the invention is that it is capable of generating signals from simple surface displacements, the processing of which makes pattern and shape recognition possible. The solution described hereinabove is very simple, and it can be configured corresponding to the requirements for resolution and sensitivity.

## Claims

1. Device for detecting the spatial pattern on surfaces and/or the shape of objects, and/or the local displacements on surfaces, and/or for slip-free gripping and lifting of an object applying an adequate compression force thereon containing at least two deformable, resilient sensor carrier layers (1), at least one deformable intermediate layer (4) and sensors (10) **characterised in that** between each two adjacent sensor carrier layers (1) an intermediate layer (4) is arranged which is more easily deformable than the adjacent sensor carrier layers (1); geometrical formations (7) are provided on the surfaces of the sensor carrier layers (1) and the intermediate layers (4) forming convex surface parts (8) on one of the surfaces of the sensor carrier layers (1) and the intermediate layers (4) and concave surface parts (9) on the other surfaces thereof, except for the external surfaces of exterior sensor carrier layers (1); each of the convex surface parts (8) of the sensor carrier layers (1) has the same shape and size as the corresponding concave surface part (9) of the adjacent sensor carrier layer (1) and the convex surface part (8) is fitted into the concave surface part (9) with the intermediate layer (4) in between; the sensors (10) are connected to the convex surface parts (8) of the sensor carrier layers (1) and to the corresponding concave surface parts (9) of the adjacent sensor carrier layers (1) in pairs for detecting the extent of the displacement between the convex surface parts (8) and the concave surface parts (9), and/or the rate of this displacement, and/or the extent of tension or compression resulting from this displacement; the sensors (10) are provided with means for carrying the output signals thereof.

2. The device according to claim 1 **characterised in that** the geometrical formations (7) are polygon-base pyramids, truncated pyramids, cones, conical frusta or hemispheres.

3. The device according to claim 1 or 2 **characterised in that** there is a sensor (10) connecting the convex surface parts (8) and the concave surface parts (9) fitted into one another at a point of the geometrical formations (7) being farthest from the plane of the corresponding sensor carrier layer (1).

4. The device according to any of claims 1-3 **characterised in that** there is at least three sensors (10) connecting the convex surface parts (8) and the concave surface parts (9) fitted into one another at points of the geometrical formations (7) other than the point being farthest from the plane of the corresponding sensor carrier layer (1).

5. The device according to claim 1 **characterised in that** the geometrical formations (7) are tetrahedrons.

6. The device according to claim 1 **characterised in that** the geometrical formations (7) are square-base pyramids.

7. The device according to claim 5 or 6 **characterised in that** there is a sensor (10) connecting the convex surface parts (8) and the concave surface parts (9) fitted into one another at the vertices of the geometrical formations (7).

8. The device according to any of claims 5-7 **characterised in that** there is at least one sensor (10) connecting the convex surface parts (8) and the concave surface parts (9) fitted into one another at each of the planes of the geometrical formations (7).

9. The device according to any of claims 1-8 **characterised in that** there are convex surface parts (8) and concave surface parts (9) formed by geometrical formations (7) of different shapes and/or sizes on the sensor carrier layers (1) and the intermediate layers (4).

10. The device according to any of claims 1-9 **characterised in that** the density of the convex surface parts (8) and the concave surface parts (9) are varying on the sensor carrier layers (1) and the intermediate layers (4).

11. The device according to any of claims 1-10 **characterised in that** the external surface of one of the exterior sensor carrier layers (1) is a contacting surface (14) for connecting to the object (3) to be touched or gripped.

12. The device according to claim 11 **characterised in that** there are bristles or ribs (5) and grooves (6) on the contacting surface (14).

13. The device according to claim 11 **characterised in that** the contacting surface (14) is formed by a smooth, soft, malleable, but elastic contacting layer (13) fixed to the exterior sensor carrier layer (1).

14. The device according to any of claims 1-13 **characterised in that** the external surface of the other exterior sensor carrier layer (1) is a base surface (15) for connecting to a support surface (2).

15. The device according to any of claims 1-14 **characterised in that** the thickness of the sensor carrier layers (1) is increased from the contacting surface (14) towards the base surface (15).

16. The device according to any of claims 1-15 **characterised in that** the stiffness of the sensor carrier layers (1) is increased from the contacting surface (14) towards the base surface (15).

17. The device according to any of claims 1-16 **characterised in that** the thickness of the intermediate layers (1) is decreased from the contacting surface (14) towards the base surface (15).

18. The device according to any of claims 1-17 **characterised in that** the viscosity of the intermediate layers (1) is increased from the contacting surface (14) towards the base surface (15).

19. The device according to any of claims 1-18 **characterised in that** the sensors (9) are Hall-sensors, potentiometers, optical sensors, magnetic sensors, capacity-type sensing elements, piezoelectric transducers or sensing elements made of conductive rubber.

20. The device according to any of claims 1-19 **characterised in that** the exterior sensor carrier layer (1) forming the contacting surface (14) contains thermal sensors (11) detecting the temperature of the object (3) being touched or gripped and/or chemical sensors (12).

## Patentansprüche

1. Gerät zum Detektieren von räumlichen Mustern auf Flächen und/oder der Form von Gegenständen und/oder von lokalen Verschiebungen auf Flächen und/oder zum schlupffreien Ergreifen und Heben eines Objektes unter Anwendung einer darauf ausgeübten adequaten Druckkraft enthaltend mindestens zwei deformierbare elastische Sensorträgerschichten (1), mindestens eine deformierbare Zwischenschicht (4) und Sensoren (10), **dadurch gekennzeichnet, dass** zwischen jeweils zwei benachbarten Sensorträgerschichten (1) eine Zwischenschicht (4) angeordnet ist, welche leichter deformierbar ist als die benachbarten Sensorträgerschichten (1); auf den Oberflächen der Sensorträgerschichten (1) und der Zwischenschichten (4) geometrische Gebilde (7) vorgesehen sind, die konvexe Oberflächenteile (8) auf einer der Oberflächen der Sensorträgerschichten (1) und der Zwischenschichten (4) und konkave Oberflächenteile (9) auf deren anderen Oberflächen mit Ausnahme der äußeren Oberflächen der äußeren Sensorträgerschichten (1) bilden; jede der konvexen Oberflächenteile (8) der Sensorträgerschichten (1) die gleiche Form und Größe wie die korrespondierenden konkaven Oberflächenteile (9) der benachbarten Sensorträgerschicht (1) aufweist und das konvexe Oberflächenteil (8) mit dazwischen eingefügter Zwischenschicht (4) in das konkave Oberflächenteil (9) eingepasst ist; die Sensoren (10) zum Detektieren des Ausmaßes der Verschiebung zwischen den konvexen Oberflächenteilen (8) und den konkaven Oberflächenteilen (9) und/oder der Geschwindigkeit dieser Verschiebung und/oder des Ausmaßes der/des aus dieser Verschiebung resultierenden Spannung oder Druckes an den konvexen Oberflächenteilen (8) der Sensorträgerschichten (1) und an den korrespondierenden konkaven Oberflächenteilen (9) der benachbarten Sensorträgerschichten (1) in Paaren angeschlossen sind; die Sensoren (10) mit einem Mittel zum Ableiten ihrer Ausgangssignale versehen sind.

2. Gerät nach Anspruch 1, **dadurch gekennzeichnet, dass** die geometrischen Gebilde (7) Pyramiden mit Polygon-Basis, Pyramidenstümpfe, Kegel, Kegelstümpfe oder Halbkugeln sind.

3. Gerät nach Anspruch 1 oder 2, **dadurch gekennzeichnet, dass** ein Sensor (10) vorgesehen ist, der die ineinander eingepassten konvexen Oberflächenteile (8) und konkaven Oberflächenteile (9) an einem solchen Punkt der geometrischen Gebilde (7) verbindet, welcher von der Ebene der korrespondierenden Sensorträgerschicht (1) am weitesten entfernt ist.

4. Gerät nach einem der Ansprüche 1 bis 3, **dadurch gekennzeichnet, dass** mindestens drei Sensoren (10) vorgesehen sind, die die ineinander eingepassten konvexen Oberflächenteile (8) und konkaven Oberflächenteile (9) an solchen Punkten der geometrischen Gebilde (7) verbinden, welche von dem am weistesten von der Ebene der korrespondierenden Sensorträgerschicht (1) entfernten Punkt abweichen.

5. Gerät nach Anspruch 1, **dadurch gekennzeichnet, dass** die geometrischen Gebilde (7) Tetraeder sind.

6. Gerät nach Anspruch 1, **dadurch gekennzeichnet, dass** die geometrischen Gebilde (7) Pyramiden mit quadratischer Basis sind.

7. Gerät nach Anspruch 5 oder 6, **dadurch gekennzeichnet, dass** ein Sensor (10) vorgesehen ist, der die ineinander eingepassten konvexen Oberflächenteile (8) und konkaven Oberflächenteile (9) an dem Scheitelpunkt der geometrischen Gebilde (7)verbindet.

8. Gerät nach einem der Ansprüche 5 bis 7, **dadurch gekennzeichnet, dass** mindestens ein, die ineinander eingepassten konvexen Oberflächenteile (8) und konkaven Oberflächenteile (9) an jeder der Flächen der geometrischen Gebilde (7) verbindender Sensor vorgesehen ist.

9. Gerät nach einem der Ansprüche 1 bis 8, **dadurch gekennzeichnet, dass** konvexe Oberflächenteile (8) und konkave Oberflächenteile (9), welche von geometrischen Gebilden (7) unterschiedlicher Form und/oder Größe gebildet sind, auf den Sensorträgerschichten (1) und den Zwischenschichten (4) vorgesehen sind.

10. Gerät nach einem der Ansprüche 1 bis 9, **dadurch gekennzeichnet, dass** die Dichte der konvexen Oberflächenteile (8) und der konkaven Oberflächenteile (9) auf den Sensorträgerschichten (1) und den Zwischenschichten (4) variiert.

11. Gerät nach einem der Ansprüche 1 bis 10, **dadurch gekennzeichnet, dass** die äußere Oberfläche einer der äußeren Sensorträgerschichten (1) eine kontaktierende Oberfläche (14) zum Verbinden mit dem zu berührenden oder zu ergreifenden Gegenstand ist.

12. Gerät nach Anspruch 11, **dadurch gekennzeichnet, dass** auf der kontaktierenden Oberfläche (14) Borsten oder Rippen (5) und Nuten (6) vorgesehen sind.

13. Gerät nach Anspruch 11, **dadurch gekennzeichnet, dass** die kontaktierende Oberfläche (14) von einer glatten, weichen, formbaren, jedoch elastischen kontaktierenden Schicht (13) gebildet ist, die an der äußeren Sensorträgerschicht (1) befestigt ist.

14. Gerät nach einem der Ansprüche 1 bis 13, **dadurch gekennzeichnet, dass** die äußere Oberfläche der anderen äußeren Sensorträgerschicht (1) eine Grundfläche (15) zum Anschließen an eine Tragfläche (2) ist.

15. Gerät nach einem der Ansprüche 1 bis 14, **dadurch gekennzeichnet, dass** sich die Dicke der Sensorträgerschichten (1) von der kontaktierenden Oberfläche (14) in Richtung zur Grundfläche (15) vergrößert.

16. Gerät nach einem der Ansprüche 1 bis 15, **dadurch gekennzeichnet, dass** sich die Steifheit der Sensorträgerschichten (1) von der kontaktierenden Oberfläche (14) in Richtung zur Grundfläche (15) vergrößert.

17. Gerät nach einem der Ansprüche 1 bis 16, **dadurch gekennzeichnet, dass** sich die Dicke der Zwischenschichten (1) von der kontaktierenden Oberfläche (14) in Richtung zur Grundfläche (15) verringert.

18. Gerät nach einem der Ansprüche 1 bis 17, **dadurch gekennzeichnet, dass** sich die Viskosität der Zwischenschichten (1) von der kontaktierenden Oberfläche (14) in Richtung zur Grundfläche (15) vergrößert.

19. Gerät nach einem der Ansprüche 1 bis 18, **dadurch gekennzeichnet, dass** die Sensoren (9) Hall-Sensoren, Potentiometer, optische Sensoren, magnetische Sensoren, kapazitive Sensorelemente, piezoelektrische Wandler oder Sensorelemente aus leitfähigen Gummi sind.

20. Gerät nach einem der Ansprüche 1 bis 19, **dadurch gekennzeichnet, dass** die die kontaktierende Oberfläche (14) bildende äußere Sensorträgerschicht (1) Temperaturfühler (11), die die Temperatur des berührten oder ergriffenen Gegenstandes (3) detektieren, und/oder chemische Sensoren (12) enthält.

## Revendications

1. Dispositif pour la détection du dessin spatial de surfaces et/ou de la forme d'objets (3) et/ou des déplacements locaux sur les surfaces et/ou pour la préhension d'objets (3) sans glissement avec une pression adéquate, contenant au moins deux couches portant de senseurs (1) élastiquement déformables, au moins une couche intermédiaire (4) déformable et des senseurs (10), **caractérisé en ce que** la couche intermédiaire (4) disposée entre toutes les deux couches portant de senseurs (1) adjacentes est plus facilement déformable que les couches portant de senseurs (1) adjacentes; les couches portant de senseurs (1) et les couches intermédiaires (4) sont divisées par des configurations géométriques (7) formant des surfaces partielles convexes (8) sur l'une des surfaces des couches portant de senseurs (1) et des couches intermédiaires (4), et des surfaces partielles concaves (9) sur les autres surfaces de ces couches, sauf sur les surfaces externes des couches portant de senseurs (1) extérieurs; chaque surface partielle convexe (8) des couches portant de senseurs (1) a la même forme et la même mesure que la surface partielle concave (9) correspondante de la couche portant de senseurs (1) adjacente, et la surface partielle convexe (8) est emboîtée dans la surface partielle concave (9) avec la couche intermédiaire (4) entre elles; les senseurs (10) détectant la mesure du déplacement entre les surfaces partielles convexes (8) et les surfaces partielles concaves (9) et/ou la vitesse du déplacement et/ou la mesure de la pression ou de la traction engendrée par le déplacement sont raccordés deux à deux aux surfaces partielles convexes (8) des couches portant de senseurs (1) et aux surfaces partielles concaves (9) correspondantes des couches portant de senseurs (1) adjacentes; les senseurs (10) sont équipés de moyens conduisant leurs signaux de sortie.

2. Dispositif selon la revendication 1, **caractérisé en ce que** les configurations géométriques (7) sont des pyramides à base polygonale, des pyramides tronquées, des cônes, des cônes tronqués ou des hémisphères.

3. Dispositif selon la revendication 1 ou 2, **caractérisé en ce qu'**un senseur (10) connectant les surfaces partielles convexes (8) et les surfaces partielles concaves (9) emboîtées est disposé à un point des configurations géométriques (7) qui se trouve le plus loins du plan de la couche portant de senseurs (1) correspondante.

4. Dispositif selon l'une des revendications 1 à 3, **caractérisé en ce qu'**au moins trois senseurs (10) connectant les surfaces partielles convexes (8) et les surfaces partielles concaves (9) emboîtées sont disposés aux points des configurations géometriques (7) qui diffèrent du point qui se trouve le plus loin du plan de la couche portant de senseurs (1) correspondantes.

5. Dispositif selon la revendication 1, **caractérisé en ce que** les configurations géométriques (7) sont des tetraèdres.

6. Dispositif selon la revendication 1, **caractérisé en ce que** les configurations géométriques (7) sont des pyramides à base carrée.

7. Dispositif selon la revendication 5 ou 6, **caractérisé en ce qu'**un senseur (10) connectant les surfaces partielles convexes (8) et les surfaces partielles concaves (9) emboîtées est disposé aux sommets des configurations géométriques (7).

8. Dispositif selon l'une des revendications 5 à 7, **caractérisé en ce qu'**au moins un senseur (10) connectant les surfaces partielles convexes (8) et les surfaces partielles concaves (9) emboîtées est disposé à chaque face des configurations géométriques (7).

9. Dispositif selon l'une des revendications 1 à 8, **caractérisé en ce qu'**il y a des surfaces partielles convexes (8) et des surfaces partielles concaves (9) formées par des configurations géométriques (7) avec des formes et/ou des mesures différantes sur les couches portant de senseurs (1) et sur les couches intermédiaries (4).

10. Dispositif selon l'une des revendications 1 à 9, **caractérisé en ce que** la densité des surfaces partielles convexes (8) et des surfaces partielles concaves (9) est variable sur les couches portant de senseurs (1) et les couches intermédiaires (4).

11. Dispositif selon l'une des revendications 1 à 10, **caractérisé en ce que** la surface externe de l'une des couches portant de senseurs (1) extérieures est une surface de contact (14) se rattachant à l'objet (3) à toucher ou à prendre.

12. Dispositif selon la revendication 11, **caractérisé en ce qu'**il y a des micropoils ou rainures (6) et des rides (5) disposés sur la surface de contact (14).

13. Dispositif selon la revendication 11, **caractérisé en ce que** la surface de contact (14) est formée d'une couche de contact (13) lisse, molle, formable mais élastique, attachée à la couche portant de senseurs (1) extérieure.

14. Dispositif selon l'une des revendications 1 à 13, **caractérisé en ce que** la surface externe de l'autre couche portant de senseurs (1) extérieure est une surface de base (15) se rattachant à une surface de support (2).

15. Dispositif selon l'une des revendications 1 à 14, **caractérisé en ce que** l'épaisseur des couches portant de senseurs (1) augmente de la surface de contact (14) vers la surface de base (15).

16. Dispositif selon l'une des revendications 1 à 15, **caractérisé en ce que** la rigidité des couches portant de senseurs (1) augmente de la surface de contact (14) vers la surface de base (15).

17. Dispositif selon l'une des revendications 1 à 16, **caractérisé en ce que** l'épaisseur des couches intermédiaires (4) diminue de la surface de contact (14) vers la surface de base (15).

18. Dispositif selon l'une des revendications 1 à 17, **caractérisé en ce que** la viscosité des couches intermédiaires (4) augmente de la surface de contact (14) vers la surface de base (15).

19. Dispositif selon l'une des revendications 1 à 18, **caractérisé en ce que** les senseurs (10) sont des senseurs Hall, des potentiomètres, des senseurs optiques, des senseurs magnétiques, des senseurs capacitifs, des transducteurs piézo-électriques ou des éléments senseurs en gomme conducteur.

20. Dispositif selon l'une des revendications 1 à 19, **caractérisé en ce que** la couche portant de senseurs (1) extérieure formant la surface de contact (14) contient des senseurs thermiques (11) détectant la température de l'objet (3) touché ou pris et/ou des senseurs chimiques (12).
